Europäisches Patentamt

European Patent Office

Office européen des brevets

(11) Publication number: **0 447 897 A2**

# EUROPEAN PATENT APPLICATION

(21) Application number: 91103616.8

(22) Date of filing: 08.03.91

(51) Int. Cl.5: **A61K 35/16, A23L 1/305,** A23J 3/12

(30) Priority: 09.03.90 JP 59668/90

(43) Date of publication of application:
25.09.91 Bulletin 91/39

(84) Designated Contracting States:
AT BE CH DE DK ES FR GB GR IT LI LU NL SE

(71) Applicant: KYOWA HAKKO KOGYO CO., LTD.
6-1, Ohtemachi 1-chome
Chiyoda-ku Tokyo 100(JP)

(72) Inventor: Yasutoshi, Takeichi
4845-4-222, Ami, Ami-machi
Inashiki-gun, Ibaraki(JP)
Inventor: Iwao, Satou
2120-11, Higashiohhashi
Ishioka-shi, Ibaraki(JP)
Inventor: Yoshinobu, Kaji
2981-10, Honmachida
Machida-shi, Tokyo(JP)
Inventor: Katsuya, Nasu
4-5-9, Mizukino, Moriya-machi
Kitasouma-gun, Ibaraki(JP)

(74) Representative: Kinzebach, Werner, Dr. et al
Patentanwälte Reitstötter, Kinzebach und
Partner Sternwartstrasse 4 Postfach 86 06 49
W-8000 München 86(DE)

(54) Dry plasma product and process for the production thereof.

(57) A dry plasma product containing not more than 0.5 % (w/w) of glucose having a high gel strength even after preserving for a long period of time at room temperature and a process for producing it.

## FIELD OF THE INVENTION

The present invention relates to a dry plasma product which contains not more than 0.5 % by weight (the same will apply hereinafter) of glucose and a process for producing it. The dry plasma product has an excellent gel strength even after preserving at room temperature for a long period of time and is useful as a material for producing processed meat products and prepared foods.

## BACKGROUND OF THE INVENTION

Plasma which sets to a gel upon heating has been employed as a binder for producing processed meat products (for example, ham, sausage) and prepared foods (for example, hamburger steak, meat balls).

Plasma is used in the form of a frozen or dry product. In particular, a dry plasma product has been conveniently used since it can be easily handled. A known process for the production of a dry plasma product includes, for example, collecting the blood of slaughtered cattle or pigs, adding a blood coagulation inhibitor to the collected blood, removing erythrocytes from the blood by centrifuging to give plasma and then drying the resulting plasma. The dry plasma product thus obtained contains approximately 1.2 % of glucose. Bovine plasma product and swine plasma product respectively contain approximately 1.2 % and 1.5 % of glucose based on solid matter [cf. "Kachiku Seikagaku" (Biochemistry for livestock, 219 - 223 (1968)].

A conventional dry plasma product has the disadvantage in that its gel strength is considerably reduced when it is stored at room temperature for a long period of time. As the result of extensive studies, it has been found that this decrease in the gel strength is caused by glucose contained in the dry plasma product.

On the other hand, it has been known that a product, formed by the Maillard reaction between glucose and proteins contained in dry egg white, causes browning of this dry egg white. It is also known that this browning can be inhibited by oxidizing glucose in the egg white with glucose oxidase. It has been known, however, that the gel strength of the dry egg white would be drastically lowered by this enzymatic treatment, compared with untreated dry egg white [cf. "Tamago - Sono Kagaku to Kako Gijutsu" (Egg - Chemistry and Process Technique Thereof), 227, ed. by Taiyo Kagaku K.K. (1985)].

It was expected that a dry plasma treated with glucose oxidase would suffer from a decrease in gel strength, similar to the case of dry egg white. However, it has been surprisingly found that the gel strength of the dry plasma product treated with glucose oxidase can be sustained for a longer period of time, compared with untreated dry plasma product.

## SUMMARY OF THE INVENTION

An object of the present invention is to provide a dry plasma product containing not more than 0.5 %-(w/w) of glucose. Another object of the present invention is to provide a process for the production of the same which comprises: (1) adding glucose oxidase to livestock plasma to oxidize glucose and drying the reaction mixture thus obtained; (2) adding to livestock plasma microbial cells, which have been obtained by inoculating a microorganism capable of metabolizing glucose into a growth medium and incubating the microorganism therein, removing the cells from the culture medium obtained by incubating the microbial cells and drying the supernatant; or (3) ultrafiltering livestock plasma and drying the concentrate thus obtained.

## DETAILED DESCRIPTION OF THE INVENTION

The livestock to be used as the source of plasma in the present invention include, for example, cows, oxen and pigs.

The livestock plasma may be obtained through conventional treatment. For example, it may be obtained by adding a blood coagulation inhibitor to livestock blood and then removing erythrocytes therefrom by centrifuging.

Examples of suitable blood coagulation inhibitors include sodium or potassium salts of citric acid, oxalic acid, phosphoric acid, sulfuric acid and polyphosphoric acid. These blood coagulation inhibitors may be employed in an amount of from 0.3 to 1.5 % based on the weight of the blood.

Step (1) of this process is described below.

As the glucose oxidase (EC.1.1.3.4), one originating from a microorganism may be used.

Although oxygen is required in the oxidation of glucose with the glucose oxidase, the reaction can proceed using the oxygen contained in the solution or the oxygen added to the solution by bubbling in the atmospheric air.

When the quantity of dissolved oxygen serves as a rate-determining factor, as the reaction proceeds, and thus restricts the progress of the reaction, it is possible to use catalase, whereby the hydrogen peroxide formed during the reaction is decomposed, so as to utilize the oxygen thus formed.

When only a small amount of oxygen is dissolved in the reaction system, oxygen may be supplied by, for example, bubbling, stirring or blowing it into the reation system. However, it is convenient and efficient to add catalase and hydrogen peroxide to the reaction system so as to utilize the oxygen thus formed.

Catalase (EC.1.11.1.6) is employed as a mixture with glucose oxidase. It is available in the form of a commercially available product, for example, DEEO® (manufactured by Miles Co., glucose oxidase activity: 1500 U/g).

Glucose oxidase having an enzymatic activity of from 1500 to 5000 U can be used per 10 kg of the livestock plasma. The reaction may be performed at a temperature of from 3 to 30 °C for 4 to 30 hours.

The reaction product may be dried by, for example, spray drying or freeze drying. The dry plasma product thus obtained preferably contains not more than 0.5 % of glucose and not more than 10 % of moisture.

Step (2) of this process is described below.

As the microorganism capable of metabolizing glucose, any microorganism may be used so long as it can metabolize glucose. For example, microorganisms belonging to the genus Saccharomyces such as Saccharomyces cerevisiae (Kyowa Dia Yeast YST®), those belonging to the genus Enterobacter such as Enterobacter aerogenes ATCC 13048 and those belonging to the genus Streptococcus such as Streptococcus lactis ATCC 15346 may be selected therefor.

As the medium in which the microorganism is incubated to obtain the desired microbial cells, either a synthetic growth medium or a natural one may be used so long as it contains the usual ingredients needed for growth, for example, a carbon source, a nitrogen source, and inorganic salts.

Examples of the carbon source include glucose, maltose and sucrose. Examples of the nitrogen source include meat extract, yeast extract and peptone. Examples of the inorganic salts include sodium chloride and monopotassium phosphate. The incubation may be at a pH value of from 5 to 8 at a temperature of from 10 to 30 °C for 60 to 150 minutes.

The cells used for the reaction are conveniently removed from the culture medium by, for example, centrifuging. After adding the cells to the livestock plasma, the culture is incubated at a pH value of from 5 to 10 at a temperature of from 0 to 45 °C for 30 minutes to 48 hours. Then the cells are separated from the culture medium by, for example, centrifuging to give a supernatant.

The supernatant may be dried in the same manner as the one described in the above process (1).

Step (3) of the process is described below.

As the ultrafiltration membrane, a synthetic resin membrane (for example, polysulfone membrane or cellulose membrane) having a fractional molecular weight of from 5,000 to 60,000 may be used. The ultrafiltration is at a flow rate of from 0.5 to 5 l/h•m² at 3 to 30 °C for 1 to 10 hours.

After completion of the ultrafiltration, the concentrate thus obtained is dried in the same manner as the one described in the above process (1).

The relation between the glucose content in the dry plasma product and the gel strength (breaking load: expressed in grams) is described with reference to the following Test Example.


TEST EXAMPLE


An ox was slaughtered. To 18 kg of the blood collected from the animal with a hollow knife, was added 90 g of sodium citrate as a coagulation inhibitor. Then the mixture was continuously centrifuged at 3,000 G to remove erythrocytes. To 10 kg of the plasma thus obtained (solid content: 11.5 %, glucose content: 1.2 % based on solid matter), was added 2 g of DEEO® (glucose oxidase activity: 3000 U). Further, 1 ml portions of a 35 % (v/v) aqueous solution of hydrogen peroxide were added thereto at the initiation of the reaction and then at intervals of 2 hours till the eighth hour of the reaction. The reaction was performed under the following conditions.

Reaction conditions:

Reaction temperature: 5 °C.

Reaction time (hr): 0, 1.5, 2.5, 5.0, 8.0 and 30.0.

After the completion of the reaction, the reaction mixture was spray dried to obtain 1.10 kg (moisture content: 4 %) of dry plasma product.

Table 1 shows the change of the glucose content of the dry plasma product during the course of the reaction.

3

## Table 1

| Sample | Enzymatic reaction time (hr) | Glucose content in dry plasma product* (%) |
|:------:|:---------------------------:|:------------------------------------------:|
| 1 | 0 | 1.20 |
| 2 | 1.5 | 0.97 |
| 3 | 2.5 | 0.80 |
| 4 | 5.0 | 0.49 |
| 5 | 8.0 | 0.23 |
| 6 | 30.0 | 0.05 |

Note:

*: Glucose content was determined with the use of a glucose determination kit (catalogue No. 716251, manufactured by Boehringer, Mannheim & Yamanouchi Co.).

Preparation of dry plasma product gel and determination of gel strength thereof

100 g of the dry plasma product was mixed with 700 g of water and then the pH value of the obtained mixture was optionally adjusted to 7. The mixture was filled in a casing made of vinylidene chloride (diameter: 3 cm) and both ends of the casing were fastened with a string. Then it was heated in a water bath at 75 °C for 30 minutes and cooled by allowing to stand in running water for 1 hour. After cooling, the casing was removed and the content was cut at intervals of 3.0 cm. The gel strength of this product was determined with the use of a rheometer NRM2002J (manufactured by Fudo Kogyo K.K.) under the following conditions.

Determination conditions:

Adapter: spherical, 1 cm in diameter.

Compression rate: 6 cm/min.

Determination range: 2 kg (maximum).

Height of sample: 3 cm.

The above-mentioned method was also employed in the following Examples.

Table 2 shows the relationship between the preservation time of the dry plasma product and the gel strength [expressed in load (g) at the breakage of the gel loaded under the conditions as specified above, i.e., breaking load] of the product obtained by the above-mentioned method.

## Table 2

| Sample | Preservation time (days) | | | | | |
|---|---|---|---|---|---|---|
| | 0 | 10 | 20 | 30 | 40 | 50 |
| 1 | 465.0 | 350.0 | 250.0 | – | – | – |
| | 305.0 | 200.0 | 180.0 | – | – | – |
| 2 | 455.0 | 350.5 | 300.0 | 250.0 | – | – |
| | 310.0 | 280.0 | 275.0 | 225.0 | – | – |
| 3 | 465.0 | 355.0 | 310.0 | 280.0 | 260.0 | – |
| | 300.0 | 290.0 | 280.0 | 223.0 | 200.0 | – |
| 4 | 460.0 | 380.0 | 350.0 | 300.0 | 280.0 | 285.0 |
| | 290.0 | 270.0 | 260.0 | 230.0 | 220.0 | 200.0 |
| 5 | 455.0 | 360.0 | 340.0 | 310.0 | 275.0 | 290.0 |
| | 310.0 | 290.0 | 255.0 | 230.0 | 210.0 | 195.0 |
| 6 | 458.0 | 375.0 | 355.0 | 320.0 | 300.0 | 315.5 |
| | 300.0 | 280.0 | 265.0 | 220.0 | 200.0 | 186.5 |

Note:

Breaking load: g

Preservation temperature: 45 °C

upper column: pH value was not adjusted.

lower column: pH value was adjusted to 7.

-: Could not determined due to low gel strength.

(The same will apply in the tables of the following Examples).

As is apparent from the results shown in Table 2, each of the samples 4 to 6 containing not more than 0.5 % of glucose in the dry plasma product showed high gel strength after preserving for a long period of time.

The present invention is further illustrated referring to the following non-limiting Examples.

## EXAMPLE 1

An ox was slaughtered. To 18 kg of the blood collected from the animal with a hollow knife, was added 90 g of sodium citrate as an anticoagulant. Then the mixture was continuously centrifuged at 3,000 G to remove erythrocytes. To 10 kg of the plasma thus obtained (solid content: 11.5 %, glucose content: 1.2 % based on solid matter), was added 2 g of DEEO®. Further, 1 ml portions of a 35 % (v/v) aqueous solution of hydrogen peroxide as an oxygen source were added thereto at the initiation of the reaction and then at intervals of 2 hours till the eighth hour of the reaction. The reaction was performed at a temperature of 5 °C for 30 hours. Then the reaction mixture was spray dried to give 1.15 kg of dry plasma product (glucose content: 0.05 %, moisture content: 4 %). This dry plasma product (the product of this invention) was preserved at 45 °C and then the gel strength was compared with that of another dry plasma product from which sugars had not been removed (control *, the same will apply hereinafter). Table 3 shows the results.

*: Dry plasma product prepared by removing erythrocytes from blood and then spray drying the obtained plasma.

EP 0 447 897 A2

## Table 3

| | Preservation time (days) | |
|---|---|---|
| Sample | 0 | 50 |
| Invention | 456 | 315 |
| | 305 | 186 |
| Control | 465 | – |
| | 305 | – |

From the results shown in Table 3 it is clear that the product of this invention has excellent storage stability as compared to the control product.

EXAMPLE 2

The procedure of Example 1 was repeated except that the plasma source was a pig to give plasma from which erythrocytes had been removed.

To 10 kg of the plasma thus obtained (solid content: 8.2 %, glucose content: 1.5 % based on solid matter), was added 2 g of DEEO®. Further, 1 ml portions of a 35 % (v/v) aqueous solution of hydrogen peroxide were added thereto at the initiation of the reaction and then at intervals of 2 hours till the tenth hour of the reaction. The reaction was conducted at a temperature of 5 °C for 30 hours.

The obtained reaction mixture was spray dried to give 1.2 kg of dry plasma product (glucose content: 0.05 %, moisture content: 4 %). The gel strength of the obtained product was then determined in the same manner as in Example 1. Table 4 shows the results.

## Table 4

| | Preservation time (days) | |
|---|---|---|
| Sample | 0 | 50 |
| Invention | 420 | 300 |
| | 300 | 180 |
| Control | 460 | – |
| | 305 | |

From the results shown in Table 4 it is clear that the product of this invention has excellent storage stability as compared to the control product.

EXAMPLE 3

10 kg of dry plasma product (solid content: 11.5 %, glucose content: 1.2 % based on solid matter), which had been obtained by removing erythrocytes in the same manner as in Example 1, was subjected to ultrafiltration.

Ultrafiltration conditions:
Device: Hollow Fiber System $CH_2$ (Grace Japan K.K.).
Ultrafiltration membrane: Polysulfone membrane having fractional molecular weight of 30,000.
Flow rate: 2.0 l/h•m$^2$.
Treatment time: 5 hrs.

The concentrate thus obtained was spray dried to give 1.13 g of dry plasma product (glucose content: 0.05 %, moisture content: 4 %).

The gel strength of the obtained product was determined in the same manner as in Example 1. Table 5 shows the results.

6

## Table 5

| | Preservation time (days) | |
|---|---|---|
| Sample | 0 | 50 |
| Invention | 445 | 305 |
| | 310 | 185 |
| Control | 460 | – |
| | 305 | – |

As shown in Table 5, it is clear that the product of this invention is excelllent in storage stability as compared to the control product.

EXAMPLE 4

To 10 kg of dry plasma product (solid content: 11.5 %, glucose content: 1.2 % based on solid matter), which had been obtained by removing erythrocytes in the same manner as in Example 1, were added 10 g (in terms of dry matter) of microbial cells* obtained by the incubation as described below. After adjusting the pH value of the mixture to 6.0 with lactic acid, these cells were incubated at a temperature of 25 °C for 120 minutes. After completion of the incubation, the culture medium was continuously centrifuged at 6,000 G to remove the cells. The supernatant thus obtained was then spray dried to give 1.15 kg of dry plasma product (glucose content: 0.05 %, moisture content: 4 %).

Method for obtaining the cells*:

A YM medium containing 4 g/l of yeast extract, 10 g/l of malt extract and 4 g/l of glucose was inoculated with Kyowa Dia Yeast YST® (manufactured by Kyowa Hakko Kogyo Co., Ltd.). Then the yeast was incubated at 25 °C for 18 hours. After completion of the incubation, the cells were collected by centrifugation.

The gel strength of the obtained product was determined in the same manner as in Example 1. Table 6 shows the results.

## Table 6

| | Preservation time (days) | |
|---|---|---|
| Sample | 0 | 50 |
| Invention | 450 | 310 |
| | 310 | 195 |
| Control | 460 | – |
| | 305 | – |

As shown in Table 6, the product of this invention is superior to the control product in storage stability.

EXAMPLE 5

To 10 kg of dry plasma product (solid content: 11.5 %, glucose content: 1.2 % based on solid matter), which had been obtained by removing erythrocytes in the same manner as in Example 1, was added 10 g (in terms of dry matter) of an equivalent mixture of microbial cells* obtained by the incubation as described below. After adjusting the pH value of the mixture to 6.5 with lactic acid, these cells were incubated at a temperature of 25 °C for 100 minutes. After the completion of the incubation, the culture medium was continuously centrifuged at 6,000 G to remove the cells. The supernatant thus obtained was then spray dried to give 1.08 kg of dry plasma product (glucose content: 0.05 %, moisture content: 4 %). The gel strength of the obtained product was then determined in the same manner as in Example 1. Table 7 shows the results.

*Method for obtaining equivalent mixture of cells:

Enterobacter aerogenes ATCC13048 and Streptococcus lactis ATCC15346 were each inoculated into a bouillon medium containing 10 g/l of peptone, 7 g/l of meat extract and 3 g/l of common salt. After incubating at a temperature of 25 °C for 18 hours, each culture medium was centrifuged. The cells thus obtained were mixed together in equal amounts to thereby give an equivalent mixture.

## Table 7

| Sample | Preservation time (days) | |
|---|---|---|
| | 0 | 50 |
| Invention | 435 | 315 |
| | 315 | 190 |
| Control | 455 | – |
| | 315 | |

As shown in Table 7, the product of this invention has excellent storage stability as compared to the control product.

## EXAMPLE 6

To 10 kg of dry plasma product (solid content: 11.5 %, glucose content: 1.2 % based on solid matter), which had been obtained by removing erythrocytes in the same manner as the one described in Example 1, was added 2 g of DEEO®. The resulting mixture was allowed to react for 30 hours while blowing air into it at a rate of 200 ml/min. The obtained reaction mixture was then spray dried to thereby give 1.15 kg of dry plasma product (glucose content: 0.05 %, moisture content: 4 %).

The gel strength of the obtained product was then determined in the same manner as in Example 1. Table 8 shows the results.

## Table 8

| Sample | Preservation time (days) | |
|---|---|---|
| | 0 | 50 |
| Invention | 445 | 315 |
| | 320 | 190 |
| Control | 450 | – |
| | 305 | |

As shown in Table 8, it is found that the invention product is superior to the control product in storage stability.

The dry plasma product of the present invention has a high gel strength even after preserving for a long period of time at room temperature.

While the invention has been described in detail and with reference to specific examples thereof, it will be apparent to one skilled in the art that various changes and modifications can be made therein without departing from the spirit and scope thereof.

**Claims**

1. A dry plasma product containing not more than 0.5 % (w/w) of glucose.

2. The dry plasma product of claim 1 containing up to 10% moisture.

3. A process for the production of a dry plasma product containing not more than 0.5 % (w/w) of glucose, which comprises adding glucose oxidase to livestock plasma to oxidize glucose and drying the reaction mixture thus obtained.

4. A process for the production of a dry plasma product containing not more than 0.5 % (w/w) of glucose, which comprises adding to livestock plasma microbial cells, which have been obtained by inoculating a microorganism capable of metabolizing glucose into a growth medium and incubating the cells therein, removing the cells from the culture medium obtained by incubating the cells and then drying the obtained supernatant.

5. A process for the production of a dry plasma product containing not more than 0.5 % (w/w) of glucose, which comprises ultrafiltering livestock plasma and drying the concentrate thus obtained.

6. A dry plasma product containing not more than 0.5% (w/w) of glucose produced by the process of claim 3.

7. A dry plasma product containing not more than 0.5% (w/w) of glucose produced by the process of claim 4.

8. A dry plasma product containing not more than 0.5% (w/w) of glucose produced by the process of claim 5.

9. A dry plasma product containing not more than 0.5% (w/w) of glucose and not more than 10% moisture produced by the process of claim 3.

10. A dry plasma product containing not more than 0.5% (w/w) of glucose and not more than 10% moisture produced by the process of claim 4.

11. A dry plasma product containing not more than 0.5% (w/w) of glucose and not more than 10% moisture produced by the process of claim 5.